(19) Europäisches Patentamt
European Patent Office
Office européen des brevets

(11) **EP 2 161 239 B1**

(12) **FASCICULE DE BREVET EUROPEEN**

(45) Date de publication et mention
de la délivrance du brevet:
**30.04.2014 Bulletin 2014/18**

(51) Int Cl.:
***B82Y 15/00*** *(2011.01)*      ***C07D 249/16*** *(2006.01)*
***C07D 498/08*** *(2006.01)*      ***G01N 33/58*** *(2006.01)*
***C07D 207/00*** *(2006.01)*      ***B82B 3/00*** *(2006.01)*
***C07D 235/00*** *(2006.01)*

(21) Numéro de dépôt: **09169655.9**

(22) Date de dépôt: **07.09.2009**

(54) **Nanocristaux semi-conducteurs**

Halbleiternanokristalle

Semiconductor nanocrystals

(84) Etats contractants désignés:
**AT BE BG CH CY CZ DE DK EE ES FI FR GB GR
HR HU IE IS IT LI LT LU LV MC MK MT NL NO PL
PT RO SE SI SK SM TR**

(30) Priorité: **08.09.2008 FR 0856030**

(43) Date de publication de la demande:
**10.03.2010 Bulletin 2010/10**

(73) Titulaire: **Commissariat à l'Énergie Atomique
et aux Énergies Alternatives
75015 Paris (FR)**

(72) Inventeurs:
- **Texier-Nogues, Isabelle
  38000 Grenoble (FR)**
- **Bernardin, Aude
  38000 Grenoble (FR)**

(74) Mandataire: **Le Coupanec, Pascale A.M.P.
Cabinet Nony
3, rue de Penthièvre
75008 Paris (FR)**

(56) Documents cités:
WO-A-00/28088         WO-A-2006/050262
WO-A-2006/115547      WO-A-2007/125429

WO-A-2008/016371      US-A1- 2006 110 782
US-A1- 2008 299 046

- VUNDYALA NEERAJA ET AL: "Biotin-functional oligo(p-phenylene vinylene)s synthesized using click chemistry" TETRAHEDRON LETTERS, vol. 49, no. 45, 28 août 2008 (2008-08-28) , pages 6386-6389, XP002531564 ISSN: 0040-4039
- LI M ET AL: "Responsive polymer-protein bioconjugates prepared by RAFT polymerization and copper-catalyzed azide-alkyne click chemistry" MACROMOLECULAR RAPID COMMUNICATIONS 20080701 WILEY-VCH VERLAG DE, vol. 29, no. 12-13, 1 juillet 2008 (2008-07-01), pages 1172-1176, XP002531190
- KOLB H C ET AL: "The growing impact of click chemistry on drug discovery" DRUG DISCOVERY TODAY, ELSEVIER, RAHWAY, NJ, US, vol. 8, no. 24, 15 décembre 2003 (2003-12-15), pages 1128-1137, XP002377521 ISSN: 1359-6446
- CODELLI JULIAN A ET AL: "Second-generation difluorinated cyclooctynes for copper-free click chemistry." JOURNAL OF THE AMERICAN CHEMICAL SOCIETY 27 AUG 2008, vol. 130, no. 34, 27 août 2008 (2008-08-27), pages 11486-11493, XP002531600 ISSN: 1520-5126
- RANJAN R ET AL: "Combination of living radical polymerization and click chemistry for surface modification" MACROMOLECULES 20070821 AMERICAN CHEMICAL SOCIETY US, vol. 40, no. 17, 21 août 2007 (2007-08-21) , pages 6217-6223, XP002531189

**EP 2 161 239 B1**

**Description**

Le domaine de l'invention est celui des nanocristaux semi-conducteurs, notamment fluorescents encore dénommés quantum dots ou boites quantiques.

La présente invention vise principalement à proposer une nouvelle méthode utile pour le greffage, notamment de molécules organiques, biomolécules polymères, dendrimères, complexes métalliques ou encore protéines, enzymes, acides nucléiques, lipides et anticorps en surface de ces nanocristaux.

Les nanocristaux semi-conducteurs sont des particules très intéressantes au regard des propriétés conférées par leur coeur semi-conducteur.

Ils sont dotés simultanément d'une fluorescence intense et d'une faible vitesse de photoblanchiment. Ils autorisent un ajustement de leur longueur d'onde d'émission *via* leur taille et/ou la composition de leur coeur semi-conducteur. Ils possèdent une bande large d'excitation qui permet d'exciter simultanément plusieurs nanocristaux à la même longueur d'onde (multiplexage facilité).

Ces propriétés, qui leur confèrent des avantages significatifs par rapport aux fluorophores organiques, sont mises à profit dans des applications très diverses.

Ainsi, les nanocristaux semi-conducteurs sont utilisés pour leurs propriétés optiques et/ou électroniques (rédox, et/ou de conduction électrique) pour la fabrication de capteurs, de cellules photovoltaïques...

Par ailleurs, ils sont largement utilisés dans le domaine de la biologie, en particulier en tant que traceurs pour l'imagerie, notamment optique, ou en tant qu'agents de photothérapie.

Les nanocristaux semi-conducteurs sont généralement synthétisés en milieu solvant organique où ils existent sous une forme stabilisée par des ligands, généralement des phosphines, des oxydes de phosphine, ou des chaînes grasses portant à leur extrémité une fonction amine, acide carboxylique, ou thiol. Ces ligands de stabilisation permettent de contrôler la croissance des nanoparticules lors de leur synthèse.

Toutefois, les nanocristaux obtenus à l'issu de ces synthèses conventionnelles ne s'avèrent pas utilisables en l'état pour de nombreuses applications.

Ainsi, pour des applications biologiques, il est souhaitable de conférer à ces nanocristaux, naturellement non hydrosolubles, une solubilité en milieu aqueux.

De plus, selon les applications considérées, il peut être nécessaire de les coupler à des molécules organiques, des complexes métalliques, des biomolécules, etc., et donc de disposer de nanocristaux porteurs de fonctions réactives propices à la réalisation de ces couplages.

Deux modes de fonctionnalisation principaux sont actuellement retenus pour accéder à des nanocristaux conformes à l'une et/ou l'autre des deux exigences précitées.

Le premier mode de fonctionnalisation repose sur un échange avec les ligands de stabilisation, c'est-à-dire les ligands organiques chélatants utilisés lors de la synthèse de nanocristaux en solvant organique. On remplace ces ligands de stabilisation par des ligands organiques chélatants bi-fonctionnels, comportant d'une part un groupement chimique présentant une affinité forte pour la surface des nanocristaux et d'autre part un groupement chimique favorisant une bonne solubilité des nanocristaux, dans l'eau ou en tampon aqueux, et qui est fonctionnalisable par une molécule d'intérêt.

Ces ligands organiques chélatants de substitution peuvent être par exemple des thiols, des anions carboxylates (issus d'acides gras en particulier), des phosphates, des phosphonates, des phosphines, des amines, des carbodithioates, tels que l'acide mercapto-acétique, l'acide oléïque, des chaînes poly(éthylèneglycol) fonctionnalisées en leur(s) extrémité(s) par ces mêmes groupements. On peut également employer des ligands présentant deux ou plusieurs de ces mêmes groupements afin de favoriser encore plus, par effet entropique, l'interaction entre le ligand et la nanoparticule, à l'image, par exemple, de l'acide dihydrolipoïque (2 fonctions thiols) et des phosphines sous forme d'oligomères. Selon une variante de réalisation, cette première couche de ligands organiques bi-fonctionnels ainsi formée peut être protégée en y greffant de façon covalente un polymère organique, sur lequel seront greffés *in fine* les molécules d'intérêt.

Le second mode de fonctionnalisation met en oeuvre, pour sa part, des molécules ou polymères amphiphiles. La partie hydrophobe du ligand ou du polymère interagit avec la partie hydrophobe des ligands de stabilisation d'origine des nanocristaux, et la partie hydrophile sert à stabiliser lesdits nano-cristaux en tampon aqueux.

La partie hydrophobe peut être par exemple constituée d'une ou plusieurs chaînes hydrocarbonées, ou de cycles aromatiques.

De tels ligands ou polymères amphiphiles peuvent être par exemple :

- des molécules amphiphiles, comme par exemple des acides gras, des chaînes grasses (en C6 et plus) fonctionnalisées par des groupes hydrophiles, tels que par exemple, amine, phosphate, sulfate....
- des polymères organiques amphiphiles, et
- des phospholipides comme par exemple la phosphatidyl éthanolamine (PE), la phosphatidylcholine (PC) ou des polymères tels que le PEG-PE/PC (PEG = poly(éthylène glycol) PEG-PE = PE fonctionnalisée par une chaîne PEG), susceptibles de s'auto-assembler en micelle.

Ces deux méthodes de fonctionnalisation, appropriées pour former une couche de solubilisation aqueuse à la surface des nanocristaux, ont donc respectivement pour avantage complémentaire de permettre l'introduction de fonctions réactives (essentiellement amines, thiols et acides carboxyliques, pour les nanocristaux commerciaux) propices au greffage ultérieur du nanocristal considéré avec un matériau annexe (protéines, enzymes, acides nucléiques, lipides, anticorps, oligosaccharides, complexes métalliques, polymères conducteurs ou pour l'optique non linéaire, surfaces, billes de silice, cellules photovoltaïques...).

Les méthodes classiques de greffage employées sur les nanocristaux semi-conducteurs et tirant profit des fonctions réactives présentes en surface de leur couche de solubilisation sont principalement les suivantes :

- couplage entre acides carboxyliques activés (souvent sous forme de NHS) et amines
- couplage entre thiols et maléimides et
- couplage entre carbonyles et oxyamines.

Ces méthodes permettent de conjuguer entre autres des protéines, acides nucléiques, anticorps, oligosaccharides, complexes métalliques, aux nanocristaux semi-conducteurs pour différentes applications.

Malheureusement, ce mode de fonctionnalisation n'est pas toujours satisfaisant. En effet, en fonction de la structure des molécules greffées, il est possible de réaliser conjointement des réactions indésirables avec les amines/thiols/acides carboxyliques présents.

Pour suppléer à cet inconvénient, des réactions de cycloaddition dites de *click chemistry* entre azotures et alcynes ont récemment été appliquées avec succès à la fonctionnalisation des nanocristaux (1).

Ainsi, *Binder et al.* rend compte d'une fonctionnalisation de nanocristaux semi-conducteurs reposant sur l'échange des ligands de stabilisation d'origine de ces nanocristaux avec des phosphines, portant soit des azotures, soit des alcynes terminaux, dans le but de réaliser consécutivement des cycloaddition 1,3-dipolaires. Ces cycloadditions sont réalisées soit par voie thermique (95 °C) dans le toluène ou par *click chemistry* dans le THF, en présence d'une base, d'un ligand et de Cu(I). Les produits de cycloaddition attendus sont obtenus dans les deux cas, et la formation des triazoles est attestée par le suivi en infrarouge de la disparition de la bande caractéristique des azotures à 2100 cm$^{-1}$.

Malheureusement, les nanocristaux semi-conducteurs obtenus par *click chemistry* perdent presque toute propriété de fluorescence. L'explication proposée est un quenching dû à la présence résiduel d'ions Cu(I). L'utilisation de la *click chemistry* classique (c'est-à-dire catalysée au Cu(I)) pour la modification des quantum dots ne permet donc pas de conserver les propriétés de fluorescence natives.

Dans le but de surmonter cet inconvénient, il a été proposé de s'affranchir de la présence du cuivre en activant les alcynes par des groupes électroattracteurs. Toutefois, ces composés sont alors susceptibles de subir des réactions de Michael et donc de conduire à la formation de produits autres que ceux attendus.

Il demeure donc un besoin d'une méthode simple et sélective pour réaliser le greffage de matériau en surface externe de nanocristaux semi-conducteurs sans affecter significativement leur pouvoir photoluminescent et plus particulièrement fluorescent.

La présente invention vise précisément à proposer un nouveau procédé de fonctionnalisation des nanocristaux semi-conducteurs conformes à ces exigences.

Ainsi, selon un de ses aspects, la présente invention concerne un procédé utile pour modifier en surface un nanocristal semi-conducteur comprenant au moins les étapes consistant à :

- disposer d'un nanocristal semi-conducteur dont la couche de revêtement organique est pourvue, en surface externe du nanocristal, d'au moins un groupement G1 réactif selon une réaction de cycloaddition de type *click chemistry,* et
- mettre en présence ledit nanocristal avec un matériau annexe, pourvu en surface d'au moins un groupement G2 complémentaire du groupement G1 au regard de ladite réaction *click chemistry,* dans des conditions propices à l'interaction desdits groupements G1 et G2,

**caractérisé en ce que** lesdits groupements G1 et G2 sont respectivement un azoture et un radical cycloalcynyle contraint ou inversement.

Au sens de la présente invention, l'expression « lesdits groupements G1 et G2 sont respectivement un azoture et un radical cycloalcynyle contraint ou inversement » signifie que le groupement G1 est un azoture et le groupement G2 un radical cycloalcynyle contraint ou que le groupement G1 est un radical cycloalcynyle contraint et le groupement G2 un azoture.

Selon un autre de ses aspects, l'invention concerne un nanocristal semi-conducteur notamment fluorescent dont la couche de revêtement organique est pourvue, en surface externe du nanocristal, d'au moins un radical cycloalcynyle contraint et réactif à l'égard d'un azoture selon une réaction de cycloaddition de type *click chemistry.*

Selon encore un autre de ses aspects, la présente invention vise un nanocristal semi-conducteur notamment fluorescent dont la couche de revêtement organique est greffée en surface à au moins un matériau, notamment un substrat et/ou au moins une molécule d'intérêt, obtenu selon le procédé d'invention.

Un tel substrat peut être notamment choisi parmi des surfaces de type métalliques (or, argent, platine ...), semi-conducteurs (silicium, germanium ...), oxydes (alumine, $SiO_2$, $TiO_2$, ...), organiques telles que par exemple plastiques, polymères ou gels.

Les inventeurs ont ainsi constaté que le choix d'un cycloalcynyle contraint à titre de groupement complémentaire d'un azoture à des fins de modification de nanocristaux fluorescents semi-conducteurs par une réaction de type *click chemistry* permet de donner satisfaction à l'ensemble des exigences précitées.

Certes, il est connu que les azotures réagissent très rapidement en présence de doubles liaisons contraintes dans les composés cycliques. En particulier, le document WO 2006/050262 tire profit de cette accélération de la réaction due à la contrainte de cycle pour réaliser une cycloaddition entre azoture et cyclooctyne à des fins de modifications chimiques de cellules dans un organisme vivant.

Le document de l'art antérieur Vundyala et al. (Tetrahedron Letters, 2008, 29:6386) décrit la formation d'oligomères oligo(p-phénylène vinylène)s (OPV) fonctionnalisés par la biotine, à partir d'une réaction de cyclo-addition catalysée au Cu(I) de type *click chemisrty*, entre un oligomère OPV fonctionnalisé alcyne et un dérivé biotine-PEG fonctionnalisé par un azoture. Il est par ailleurs précisé la possibilité de greffer par les sites de liaison de la biotine le composé obtenu à un quantum dot enrobé de streptavidine à des fins de conduire à un réseau de quantum dots liés entre eux par des OPVs.

Ainsi ce document est totalement silencieux quant à la mise en oeuvre d'un cycloalcyne contraint à titre de groupement complémentaire d'un azoture, conformément au procédé de la présente invention et ce, pour la résolution du problème technique de l'invention, à savoir l'obtention de nano-cristaux fluorescents greffés en surface à un substrat ou à une molécule d'intérêt, qui demeurent dotés d'une fluorescence satisfaisante.

Toutefois, à la connaissance des inventeurs, cette cycloaddition de type *click chemistry* d'un azoture sur un cycloalkyne contraint n'a jamais été mise à profit pour la modification en surface de nanocristaux fluorescents. De même, la fonctionnalisation de la couche de revêtement organique en surface de nanocristaux avec au moins un radical cycloalcynyle contraint n'a jamais été considérée pour permettre l'obtention d'une manière simple et douce de nanocristaux fluorescents greffés en surface soit à un substrat soit à une molécule d'intérêt, et qui demeurent néanmoins dotés d'une fluorescence satisfaisante.

Le procédé faisant l'objet de l'invention s'avère avantageux à plusieurs titres.

Tout d'abord, il permet la conjugaison de molécules d'intérêt, ou de biomolécules, à la surface de nanocristaux semi-conducteurs notamment fluorescents, en conditions douces et biocompatibles.

L'absence de base permet de maintenir un pH qui assure la stabilité des nanocristaux en phase aqueuse.

L'absence de cuivre(I) permet d'éviter le phénomène d'extinction de la fluorescence des nanocristaux semi-conducteurs fluorescents.

Il n'est plus nécessaire d'ajouter des additifs, tels que des polytriazoles qui permettent une autocatalyse de la réaction en stabilisant le cuivre(I).

Enfin, la fonctionnalisation peut être réalisée en milieu aqueux et à température ambiante, soit des conditions particulièrement appréciées sur le plan industriel, et par ailleurs qui s'avèrent donc compatibles avec le greffage de molécules d'intérêt fragiles, comme les acides nucléiques et certaines protéines.

D'autres caractéristiques et avantages de l'invention ressortiront mieux de la description qui suit.

## Nanocristaux semi-conducteurs

Les nanocristaux peuvent être définis comme des objets cristallins « nanométriques », c'est-à-dire que leur taille est généralement inférieure à 150 Å et de préférence est comprise dans la plage de 12 à 150 Å.

Les nanocristaux considérés selon l'invention sont des nanocristaux inorganiques à couche de revêtement organique.

La taille du coeur cristallin inorganique est généralement inférieure à 100 nm, et de préférence compris entre 1 et 25 nm.

Les nanocristaux semi-conducteurs sont généralement constitués par au moins un métal, un oxyde métallique et/ou au moins un composé semi-conducteur.

Les nanocristaux sont par exemple constitués par au moins un métal.

Le métal peut être quelconque, mais il est généralement choisi parmi les métaux de transition, les métaux des terres rares, les métaux des groupes IIIA, IVA et VA du tableau périodique des éléments, et leurs alliages, et les mélanges de ces métaux et alliages.

De préférence, le métal est choisi parmi l'aluminium, le cuivre, l'argent, l'or, l'indium, le fer, le platine, le nickel, le molybdène, le titane, le tungstène, l'antimoine, le palladium, le zinc, l'étain, leurs alliages, et mélanges de ces métaux et alliages.

De préférence, le métal est l'or.

Les nanocristaux peuvent être également constitués par au moins un oxyde métallique, comme par exemple un oxyde de fer, de titane, d'aluminium, de platine, de gadolinium ou d'hafnium. De préférence, les nanocristaux sont des oxydes de fer dont en particulier la magnétite, maghémite, l'oxyde de gadolinium ou l'oxyde de hafnium.

Les nanocristaux peuvent être constitués par au moins un composé semi-conducteur. Le composé semi-conducteur peut être un semi-conducteur de formule AB dans laquelle A représente un métal ou un métalloïde à l'état d'oxydation +II et B représente un élément à l'état d'oxydation -II.

A est choisi généralement parmi Mg, Ca, Sr, Ba, Zn, Cd, Hg, Sn, Pb et les mélanges de ceux-ci, et B est choisi généralement parmi O, S, Se, Te et les mélanges de ceux-ci.

Des exemples de composés A(II)B(VI) sont MgS, MgSe, MgTe, CaS, CaSe, CaTe, SrS, SrSe, SrTe, BaS, BaSe, BaTe, ZnO, ZnS, ZnSe, ZnTe, CdS, CdSe, CdTe, HgS, HgSe, HgTe, SnS, SnSe, SnTe, PbS, PbSe, PbTe et les mélanges de ceux-ci.

Le composé semi-conducteur peut être aussi un semi-conducteur de formule CD (C(III) D(V)) dans laquelle C représente un métal ou un métalloïde à l'état d'oxydation +III et D représente un élément à l'état d'oxydation -III.

C est choisi généralement parmi Ga, In, et leurs mélanges, et D est choisi généralement parmi Sb, As, P, N et leurs mélanges.

Des exemples de ces composés semi-conducteurs C(III)D(V) sont GaAs, GaSb, GaN, InGaAs, InN, InGaN, InP, InAs, InSb et leurs mélanges.

Il est également possible d'utiliser des composés semi-conducteurs du groupe IV tel que le silicium ou le germanium.

On peut également utiliser un mélange des composés AB, CD et de semi-conducteurs du groupe IV.

Dans une forme de réalisation de l'invention, le nanocristal semi-conducteur a une structure coeur/coquille(s) ("core/shell" en anglais), ledit coeur étant constitué par un nanocristal tel que décrit ci-dessus constitué d'au moins un métal et/ou au moins un composé semi-conducteur tandis que la(les) coquille(s) est(sont) chacune constituée(s) d'une couche d'un métal et/ou d'au moins un composé semi-conducteur comprenant au moins un métal.

Ce coeur a par exemple un diamètre de 10 à 250 Å tandis que la (les) coquille(s) a (ont) une épaisseur de 3 à 30 Å. Lorsque le nanocristal ne comporte pas de coquille(s), il a généralement un diamètre de 15 à 150 Å.

La couche de revêtement organique a généralement une épaisseur de 5 à 100 Å.

Toutes les combinaisons sont possibles pour les matériaux formant le coeur d'une part et la(les) coquille(s) d'autre part, mais, de préférence, le coeur est en un premier composé semi-conducteur, tandis que la coquille entourant ledit coeur (cas d'une seule coquille) ou la première coquille contenant le coeur dans le cas où le coeur est entouré de plusieurs coquilles, est en un second composé semi-conducteur différent du premier composé semi-conducteur (formant le coeur).

Les premier et second composés semi-conducteurs sont choisis parmi les composés semi-conducteurs déjà décrits plus haut.

De préférence, le coeur est en un premier composé semi-conducteur de type A(II)B(VI) décrit plus haut, tel que CdSe ou un composé C(III) D(V) décrit plus haut tel que InP, tandis que la coquille entourant le coeur ou la première coquille entourant le coeur, est en un second composé semi-conducteur de type A(II)B(VI) différent du premier composé semi-conducteur choisi par exemple parmi ZnSe, ZnS et CdS.

Dans le cas des multiples coquilles, deux coquilles successives sont généralement en des composés semi-conducteurs différents.

Ainsi, dans le cas des multiples coquilles, les matériaux formant les coquilles peuvent être choisis parmi toutes les combinaisons possibles des composés cités précédemment par exemple ces composés peuvent être choisis parmi ZnSe, CdS et ZnS. Par exemple, on pourra avoir une première coquille en ZnSe ou en CdS et une deuxième coquille en ZnS.

Comme précisé précédemment, les nanocristaux considérés selon l'invention possèdent une couche de revêtement organique de solubilisation spécifique.

## Couche de revêtement organique

Les nanocristaux semi-conducteurs considérés selon l'invention possèdent une couche de revêtement organique modifiée car pourvue, en surface externe du nanocristal, d'au moins un groupement G1 réactif selon une réaction *click chemistry.*

Plus précisément, cette couche comprend au moins un ligand spécifique répondant à la formule générale (I) :

(I)    L-X-E-A-G

dans laquelle :

- L est un ligand chélatant, notamment pouvant être choisi parmi la phosphine, des oxydes de phosphine, ou des chaînes grasses portant à leur extrémité une fonction amine, acide carboxylique, thiol, un groupe 1,1-dithiolate (-C (S) S$^-$) ou un groupe 1,1-disélénoate (-C (Se) Se$^-$).
- E est un groupe espaceur organique, qui peut permettre un transfert de charge ou bien être isolant,
- X est le produit réactionnel d'une fonction réactive présente sur le ligand L avec une fonction réactive complémentaire présente sur le groupe espaceur organique E telles que, par exemple, thiol/maléimide, amine/acide carboxylique activé ou carbonyle/oxyamine.
- A représente une liaison simple ou un groupe choisi parmi -CONH-, -NHCO-, -OCH$_2$CONH-, -NHCOCH$_2$O-, -O-, -S-, et
- G est un radical azoture ou cycloalcynyle contraint.

Au sens de la présente invention, le terme « cycloalcynyle contraint » s'étend aux hétérocycloalcynyles. Plus particulièrement, il s'agit d'un cycle à 7, 8 ou 9 chaînons.

Selon un mode préféré de l'invention, le groupe cycloalcynyle est un groupe cyclooctynyle.

La contrainte appliquée sur le groupe cycloalcynyle peut être accrue de diverses manières, par exemple par l'utilisation d'hétéroatomes, le degré d'insaturation, ou la déformation due à la torsion, l'utilisation de groupes électroattracteurs, etc. Les dérivés correspondants sont également couverts sous le terme « cycloalcynyle contraint ».

Ainsi, le cycloalcynyle contraint conforme à l'invention peut être un composé dans lequel un ou plusieurs atome(s) de carbone du cycle cycloalcynyle, à l'exclusion des deux atomes de carbone joints par une triple liaison, est substitué par un ou plusieurs groupe(s) électroattracteur(s), par exemple un groupement halo (bromo, chloro, fluoro, iodo), nitro, un groupement cyano ou un groupement sulfone. Lorsque le groupe électroattracteur est un groupe nitro, cyano ou sulfone, le groupe électroattracteur n'est pas directement lié au cycle cycloalcynyle.

Comme autre groupe susceptible d'être présent sur le cycloalcynyle, on peut citer, de manière non exhaustive, les groupes carboxyle, amine, (par exemple alkyl amine (par exemple alkyl amine inférieur), aryl amine), ester (par exemple alkyl ester (par exemple alkyl ester inférieur, benzyl ester), aryl ester, aryl ester substitué), thioester, sulfonyl halide, alcool, thiol, ester succinimidyle, isothiocyanate, iodoacétamide, maleimide, hydrazine, etc.

Selon une variante préférée, E répond à la formule suivante :

$$-R_1-R-\qquad\text{(II)}$$

où R$_1$ représente :

- une liaison simple ;

- un groupe

$$-\overset{\displaystyle R_2}{\underset{\displaystyle R_3}{\overset{|}{\underset{|}{C}}}}-$$

où R$_2$ et R$_3$ représentent indépendamment un hydrogène, un radical alkyle, un radical aryle, un radical alcoxy, un halogène ;

- un groupe

$$\begin{array}{c} R' \\ | \\ -N- \end{array}$$

où R' représente un hydrogène, un radical alkyle, un radical aryle, un radical alcoxy, un halogène ;

- un groupe

$$\begin{array}{c} R' \\ | \\ -P- \end{array}$$

où R' a la signification déjà donnée plus haut ;

- un groupe

$$\begin{array}{c} -P-O \\ | \\ O \\ | \\ R' \end{array}$$

où R' a la signification déjà donnée plus haut ;

- -O- ;

- -S- ;

- -Se- ;

et R représente un groupe alkylène linéaire ou ramifié ayant de 1 à 30 atomes de carbone, de préférence de 1 à 8 ; un ou plusieurs atomes de carbone dudit groupe alkylène étant éventuellement remplacés par un ou plusieurs hétéroatomes choisis parmi O, N, S, P et Si ; ledit groupe alkylène comprenant en outre éventuellement une ou plusieurs doubles et/ou triples liaisons telles que des doubles et/ou triples liaisons carbone-carbone ; et ledit groupe alkylène étant en outre éventuellement substitué par un ou plusieurs groupes choisis parmi les halogènes, tels que le chlore, le brome, l'iode et le fluor, les hétérocycles, les radicaux aryle, hydroxyle, alcoxy, amino, acyle, carboxamido ; =O, -CHO, -CO$_2$H, -SO$_3$H, -PO$_3$H$_2$, -PO$_4$H$_2$, -NHSO$_3$H, sulfonamide ; monoalkylamino, trialkylammonium, ou bien encore par un radical dialkylamino, dans lequel les deux groupements alkyle peuvent former, conjointement avec l'atome d'azote dudit groupement dialkyl(C$_1$-C$_4$)amino, auquel ils sont liés, un cycle pouvant être interrompu par un ou plusieurs atomes d'azote, d'oxygène ou de soufre, et les groupes Z ; ou R représente un hétérocycle, un radical aryle, un radical aryle condensé sur un ou plusieurs autres cycles aryles et/ou cycles alkyles ou hétérocycles, un radical cycloalkyle, un radical cycloalkyl-alkyle, un radical alkylcycloalkyle, un radical arylalkyle, un radical alkylaryle, un radical hétérocycloalkyle, un radical alkyl-hétérocycloalkyle.

Le groupe E peut être avantageusement une chaîne poly(ethylène) glycol (PEG) permettant d'améliorer la solubilité du composé, ou tout autre polymère, dendrimères, oligomères, hydrogels permettant par exemple d'encapsuler des nanocristaux semi-conducteurs fluorescents dans des matériaux, gels, ou formulation (matériaux plastiques pour l'optique et l'électronique, délivrance d'agents de photothérapie, par exemple).

Le groupe E peut également être une petite molécule organique permettant d'apporter la fonction réactive complémentaire, par exemple l'éthylène diamine, l'acide glycolique, la cystéamine, ou tout autre molécule organique permettant d'apporter des acides/amines/thiols.

Le groupe E peut être un groupe hydrocarboné comprenant une ou plusieurs insaturations, par exemple, du type alcénique. Un exemple d'un tel groupe peut être un groupe alkylène tel que défini ci-dessus interrompu par une ou plusieurs insaturations alcéniques. Lorsque le groupe E comporte au moins deux insaturations, il peut conférer aux composés, une capacité à se réticuler.

Le groupe E peut être également un groupe hydrocarboné comprenant un ou plusieurs groupes aromatiques. On peut citer, par exemple, un groupe comprenant des groupes aromatiques conjugués avec des groupes linéaires insaturés, tel qu'un groupe résultant de l'enchaînement d'un motif phénylène-vinylène. Ces groupes contribuent à conférer des propriétés optiques non linéaires.

On peut également citer un groupe comprenant des motifs pyrrole et/ou thiophène. Ces groupes contribuent à conférer au matériau des propriétés des conductions électroniques. On peut citer, par exemple, un groupe comprenant un ou plusieurs aromatiques substitués par un ou plusieurs groupes aromatiques, tel qu'un groupe comprenant un enchaînement de motifs quinones ou de motifs diazoïques. Ces groupes contribuent à conférer au composé les possédant des propriétés de photo/électroluminescence.

L'obtention des précédents nanocristaux destinés à être mise en oeuvre dans la première étape du procédé selon l'invention relève clairement des compétences de l'homme de l'art.

Selon une première variante d'obtention, les nanocristaux déjà pourvus en surface d'une couche de ligands chélatants mais non conformes à l'invention sont mis en présence, généralement en solution, avec des composés ligands déjà fonctionnalisés avec au moins un radical azoture ou cycloalkynyle contraint et l'ensemble est maintenu en contact pendant une durée satisfaisante pour réaliser à la surface des nanocristaux un échange au moins en partie des ligands chélatants d'origine avec les composés ligands fonctionnalisés avec au moins un radical azoture ou cycloalkynyle contraint.

Selon une seconde variante, il peut être directement procédé à la fonctionnalisation des ligands chélatants formant la couche externe de nanocristaux avec les motifs cycloalkynes contraint ou azoture.

Les matériaux comprenant un azoture convenant à la présente invention, tout comme les méthodes de préparation de matériaux comprenant l'azoture convenant à la présente invention, sont également bien connus de l'homme de l'art.

### Procédé

La présente invention propose donc un procédé utile pour modifier en surface des nanocristaux *via* une réaction de cycloaddition [3+2] de type *click chemistry* entre une entité azoture et une entité cycloalkynyle contrainte, l'une de ces deux entités, de préférence le cycloalkynyle contraint, étant présente en surface externe de la couche de revêtement organique des nanocristaux à greffer et l'autre étant liée à un matériau annexe destiné à être fixé en surface des nanocristaux.

Comme il ressort de ce qui précède, l'invention tire profit de l'importante déformation de l'angle de liaison du groupe acétylène dans le groupement cycloalkynyle contraint, qui assure la tension du cycle. Cette déstabilisation de l'état fondamental *versus* l'état transitionnel procure à la réaction une accélération très importante en comparaison avec les alcynes non contraints.

Le groupement azoture et le groupement cycloalkynyle contraint portés respectivement par chacun des matériaux en présence, à savoir le nanocristal ou le matériau annexe, interagissent pour former un produit final conjugué comprenant un cycle fusionné azoture/cycloalkyne.

La réaction est avantageusement obtenue sans catalyseur.

Elle peut être réalisée avantageusement à température ambiante et en milieu aqueux. L'énergie d'activation nécessaire à la réaction est fournie par le groupement azoture et le groupement cycloalcynyle contraint.

Le procédé selon l'invention peut comprendre à l'issue de sa seconde étape, une étape supplémentaire au cours de laquelle les nanocristaux pourvus d'une couche de revêtement organique modifiée selon l'invention (I) (lesdits nanocristaux ayant été éventuellement précipités, séparés, lavés, puis séchés) sont soumis à une irradiation par une lumière, de préférence une lumière ultraviolette (UV).

En d'autres termes, les nanocristaux sont soumis à une exposition à de la lumière, de préférence de la lumière ultraviolette pendant une durée donnée, généralement de une ou quelques minutes à une ou plusieurs heures par exemple de une minute à 10 heures.

Cette irradiation ou exposition, entraîne un processus photochimique qui améliore l'efficacité de la photoluminescence.

### Greffage en surface des nanocristaux

Au sens de la présente invention, le « greffage » couvre les deux réactions qui suivent, à savoir :

- l'« ancrage » qui se réfère à l'immobilisation d'un composé, en l'occurrence un nanocristal en surface d'un substrat, et
- le terme « couplage » qui décrit la réaction entre au moins un groupe fonctionnel terminal d'un premier composé, notamment un nanocristal, et au moins un groupe fonctionnel complémentaire, porté par un second composé, notamment une molécule d'intérêt.

Selon une variante de réalisation, le procédé selon l'invention est mis en oeuvre à des fins d'ancrage de nanocristaux en surface d'un matériau de type substrat.

Par exemple, un support solide ou semi-solide, en particulier un support convenant pour une utilisation en tant que puce à acides nucléiques (ADN, ARN, oligonucléotides), à protéine, à sucres, à cellules ou en tant que photosensibilisateur pour les cellules photovoltaïques, ou que marqueur optique de billes de silice (pour les puces en suspension).

Le procédé selon l'invention comprend généralement une mise en présence d'un groupement azoture présent sur le substrat avec un groupe cycloalkynyle contraint, présent au niveau du nanocristal.

Selon une autre variante de réalisation, le procédé selon l'invention est mis en oeuvre pour coupler la surface externe des nanocristaux semi-conducteurs avec une ou plusieurs molécules d'intérêt.

Comme exemple de molécules d'intérêt, on peut également citer les marqueurs, les colorants, les fluorophores (coumarines, fluorescéine, fluorescines modifiées, rhodanines, iguanines, boron-dipynométhène, oxazines et autres), les toxines (y compris les cytotoxines), les linkers, les actifs thérapeutiques, cosmétiques, phytosanitaires, les membres d'une paire liante spécifique, les peptides, les aminoacides et les résidus d'aminoacide, les polypeptides (y compris les peptides et les protéines), les sucres et les résidus du sucre, les photosensibilisateurs tels que par exemple l'éosine, le rose de bengal, les phtalocyanines, les chlorines, les bactériochlorines, les porphyrines telles que par exemple la tétrakis [;méso(4-éthynylphényl)porphyrine, la 1,1 0-(4-éthynylphényl)5,15-(4-mésityl)porphyrine et la 1-(4-éthynylphényl)5,10,15-(4-mésityl)porphyrine) dont la présence sur une surface est particulièrement utile dans le domaine de l'électronique moléculaire et de la photothérapie ; des composés présentant une isomérie cis-trans tels que les dérivés des diaryléthylènes, des spiropyrannes, des spiroxazines, des fulgides ou de l'azobenzène dont la présence sur une surface est particulièrement utile pour la fabrication d'interrupteurs moléculaires photocontrôlés.

Les molécules d'intérêt peuvent être d'origine naturelle, ou produites de manière synthétique ou recombinante, et peuvent être isolées, purifiées, ou encore présentes dans le milieu natif de la molécule non modifiée sur laquelle se base la molécule cible comprenant généralement un azoture, par exemple, à la surface ou à l'intérieur d'une cellule.

Lorsque la molécule cible est un polypeptide, le polypeptide peut être composé d'aminoacides D, L, ou des deux, et peut en outre être modifié, que ce soit de manière naturelle, synthétique, ou recombinante, afin d'y inclure d'autres groupements. Par exemple, le polypeptide cible peut être une lipoprotéine, une glycoprotéine, ou tout autre protéine modifiée.

Les procédés et nanocristaux selon l'invention ont de multiples applications, que ce soit en recherche ou en diagnostique.

Les applications de recherche comprennent également la découverte d'un médicament ou des applications de dépistage/criblage : comme tout agent de contraste, l'invention permet la visualisation de molécules d'intérêt (récepteur, glucose, antigène ...) ou de mécanismes biologiques (endocytose, néoangiogenèse, apoptose, activités enzymatiques ...) et permet donc l'identification de cibles thérapeutiques.

Elle permet aussi de faire un suivi de l'effet d'un traitement médicamenteux (par exemple cytotoxique, inhibiteur d'une enzyme ...) ou la détection de tumeurs (par l'intermédiaire de marqueurs spécifiques).

Comme autres applications d'intérêt, on peut citer l'étude des caractéristiques fonctionnelles et physiques d'un récepteur, la protéomique, la métabolomique, etc.

Références bibliographiques

(1) Binder, W. H.; Sachsenhofer, R.; Straif, C. J.; Zirbs, R., Journal of Materials Chemistry2007, 17, (20), 2125-2132

Les exemples et figures qui suivent sont présentés à titre illustratif et non limitatif au domaine de l'invention.

Figure 1 : Elle rend compte des rendements quantiques des QDots modifiés par *click chemistry* considérés en exemple 2.

Figure 2 : Elle illustre le principe de l'incorporation métabolique de l'Ac$_4$ManNAz et sa transformation en acide sialique porteur du groupe N$_3$, lequel se trouve ensuite incorporé dans les protéoglycanes exprimés sur les membranes cellulaires et permet le marquage dans une deuxième étape par une réaction click avec les QDots-cyclooctynes.

**EXEMPLES**

**Exemple 1 :**

**Synthèse et caractérisations d'un nanocristal fluorescent modifié par un motif clooctynyle**

1. Synthèse du clooctyne

a) Synthèse du 8,8-dibromobicyclo[5.1.0]octane

Dans un ballon sec et sous argon sont introduits 3,65 g de cycloheptène (soit 38 mmol) puis 8,52 g de *t*-BuOK (soit 76 mmol, 2 éq.) et 9 mL de pentane distillé au préalable. La solution jaune crème est agitée vigoureusement et placée dans un bain glace/sel. Puis 4,9 mL de bromoforme (soit 57 mmol, 1,5 éq.) sont ajoutés goutte à goutte. Lors des premiers ajouts, un dégagement gazeux assez violent est observé, puis au fur et à mesure de l'addition la solution devient ocre brune. Au cours de l'addition, environ 5 mL de pentane ont été rajoutés pour permettre une agitation correcte de la solution. Une fois l'addition terminée, le mélange est laissé revenir à température ambiante toute la nuit, sous argon et agitation vigoureuse.
Environ 50 mL d'eau sont ensuite ajoutés et le pH neutralisé avec HC1 1M. Les phases organique et aqueuse sont séparées ; la phase aqueuse est extraite avec 3x20 mL de pentane et la phase pentane est lavée avec 3x20 mL d'eau. La phase organique est ensuite séchée sur $MgSO_4$ et le solvant évaporé sous vide. Une huile jaune orangée est obtenue avec une masse m = 10,814 g.
Le produit est ensuite purifié par une simple filtration sur silice avec comme éluant cyclohexane/AcOEt 5 %. Une huile incolore de masse totale 9,100 g est obtenue après purification, soit avec un rendement de 90 %.

b) Synthèse du 2-bromo-clooctène-3-glycolate de méthyle

A une solution de 8,8-dibromobicyclo[5.1.0]octane (2,5 g soit 9,3 mmol) et de glycolate de méthyle (6,35 mL soit 83,9 mmol) dissous dans 5 mL de toluène anhydre dans un ballon sec, sous Ar et protégé de la lumière par un film d'aluminium, sont ajoutés 3,85 g de perchlorate d'argent (soit 18,6 mmol). La réaction est agitée pendant 1 h 30 à température ambiante, puis les sels d'argent sont filtrés sur fritté et lavés avec AcOEt. La solution est concentrée sous vide pour donner une huile visqueuse brune qui est purifiée par chromatographie sur gel de silice (2-15 % AcOEt dans le cyclohexane) pour obtenir le produit sous forme d'huile jaune de masse m= 1,6 g, soit 65 % de rendement.

c) Synthèse de l'acide clooct-1-yn-3-glycolique

A 250 mg de 2-bromo-clooctèn-3-glycolate de méthyle (soit 0,90 mmol) est ajoutée une solution de méthylate de sodium à 0.5M dans le méthanol. Le mélange est agité pendant 2 jours à température ambiante.
La réaction est acidifiée avec HCl 1M puis extraite avec AcOEt, séchée sur $MgSO_4$ et les solvants sont évaporés. Le produit est purifié sur gel de silice avec AcOEt et est obtenu sous forme d'huile jaune de masse 120 mg, soit 80 % de rendement.
RMN $^1$H (CDCl$_3$, 200 MHz) : $\delta$(ppm) 1.3-2.3 (m, 10H) ; 4.45 (d, $J_{9\text{-}9'}$ = 17 Hz, 1H, $H_9$) ; 4.50 (m, 1H, $H_3$) ; 4.58 (d, $J_{9\text{-}9'}$ = 17 Hz, 1H, $H_{9'}$) ;
RMN $^{13}$C (CDCl$_3$, 50 MHz) : $\delta$ (ppm)
Masse : ESI$^+$ m/z

2. Synthèse du nanocristal modifié aminoPEG-clooctyne

Le nanocristal de départ est le quantum dot commercialisé par la société Invitrogen sous la dénomination QDots ITK545.

Avant réaction, les QDots ITK 545 sont transférés du tampon borate de stockage dans le **PBS** (phosphate buffered saline, 0,01 M avec 0,154 M de NaCl à pH 7,4)

Pour cela 75 $\mu$L de solution à 8 $\mu$M sont filtrés sur des filtres Microcon® pendant 5 min à 5000 g (par centrifugation), puis rincés avec le même volume de PBS par centrifugation 5 min à 3000 g, et resuspendus dans 75 $\mu$L de PBS (1000 g pendant 3 min, filtre retourné).

0.5 mg d'**EDC** (N-(3-diméthylaminopropyl)-N'-éthylcarbodiimide) et 10 $\mu$L d'une solution de cyclooctyne dans le DMSO (0.11 mg soit 10 équivalents par fonction, 100 fonctions par nanoparticule) sont ajoutés dans les 75 $\mu$l de PBS contenant le QDots ITK545. Le tout est agité à température ambiante, à l'abri de la lumière pendant 5 h.

Le produit est ensuite purifié sur colonne d'exclusion stérique GL25 (Healthcare GE), préalablement équilibrée avec PBS. Le produit est récupéré en une fraction qui est ensuite concentrée sur filtres Microcon® à 3000 g pendant 5 min, et re-suspendue dans 75 $\mu$M de PBS à 1000 g pendant 3 min.

Une électrophorèse réalisée sur gel d'agarose à 1 %, dans un tampon TBE pH 8.3 (dépôt de 10 $\mu$L d'une solution diluée au 10ème), 20 min à 100 V permet de mettre en évidence la modification de la surface des nanocristaux ; en effet le composé modifié par des groupes cyclooctynes migre différemment en comparaison avec le produit de départ.

De même, des spectres d'absorption enregistrés à 488 nm sur un spectrophotomètre CARY 300 scan de Varian, et des spectres de fluorescence enregistrés à 488 nm sur un spectrophotomètre LS50B de Perkin Elmer montrent que les propriétés optiques des QDots ne sont pas modifiées par le couplage des groupes cyclooctynes en surface.

Le rendement quantique est de 108 % par rapport au produit de départ, mesuré dans le PBS.

## Exemple 2 :

### Comparaison du greffage d'un sucre sur des Qdots-alcynes linéaires et des QDots-cyclooctynes préparés selon l'exemple 1

1. Synthèse et caractérisation des QDots-alcynes linéaires

Avant réaction, les QDots ITK 525 aminoPEG d'Invitrogen sont transférés du tampon borate de stockage dans le PBS : pour cela 60 $\mu$L de solution à 8 $\mu$M sont filtrés sur des filtres Microcon® pendant 5 min à 5000g (par centrifugation), puis rincés avec le même volume de PBS par centrifugation 5 min à 3000 g, et re-suspendus dans 60 $\mu$L de PBS (1000 g pendant 3 min, filtre retourné).

0.5 mg d'EDC et 5 $\mu$L d'une solution d'acide undécynoïque dans le DMSO (0.087 mg soit 10 équivalents par fonction, 100 fonctions par nanoparticule) y sont ajoutés. Le tout est agité à $T_a$, à l'abri de la lumière pendant 1 h. Puis le produit est purifié sur colonne d'exclusion stérique G25 (Healthcare GE), préalablement équilibrée avec PBS. Le produit est récupéré en une fraction qui est ensuite concentrée sur filtres Microcon® à 3000 g pendant 5 min, et re-suspendue dans 60 $\mu$M de PBS à 1000 g pendant 3 min.

Les QDots ont été caractérisés par électrophorèse sur gel et leurs propriétés optiques déterminées selon le protocole décrit en exemple 1.

2. Comparaison du greffage d'un sucre sur QDots-alcynes linéaires et QDots-cyclooctynes

Le couplage d'un sucre portant un azoture a été réalisé parallèlement sur les QDots-alcynes linéaires et sur les QDots-cyclooctynes synthétisés selon les deux schémas réactionnels respectifs suivants.

- Couplage click classique entre QD-alcynes linéaires et Ac$_4$ManNAz :

A une solution de 20 % L de QDots dans PBS sont ajoutés 5 μL d'une solution d'Ac$_4$ManNAz dans le DMSO (10 éq. par fonction réactive) et 2 μL d'une solution CuSO$_4$ 1 mM/ascorbate de sodium 5 mM (0.1 éq.). Le tout est agité à température ambiante pendant 3h, puis les QD purifiés sur filtres Microcon par rinçage au PBS (1 rinçage ou 5 rinçages) à 3000 g pendant 3 minutes. Les Qdots sont ensuite caractérisés par électrophorèses sur gel et leurs propriétés optiques déterminées.

- Couplage entre QD-cyclooctyne et Ac$_4$ManNAz :

A une solution de QDots dans PBS (20 μL) est ajoutée une solution d'Ac$_4$ManNAz dans le DMSO (5μL, 10 éq. Par fonction réactive). Puis le tout est agité à température ambiante pendant 3h ; puis les QD purifiés sur filtres Microcon par rinçage au PBS (1 rinçage) à 3000 g pendant 3 minutes. Les Qdots obtenus sont ensuite caractérisés par électrophorèse sur gel et leurs propriétés optiques déterminées.

Les produits de couplage obtenus sont caractérisés en terme de spectre de fluorescence selon le protocole décrit en exemple 1, et il apparaît clairement que le rendement quantique des QDots-alcynes linéaires est très fortement diminué, alors que celui des QDots-cyclooctyne modifiés reste élevé par rapport au produit de départ.

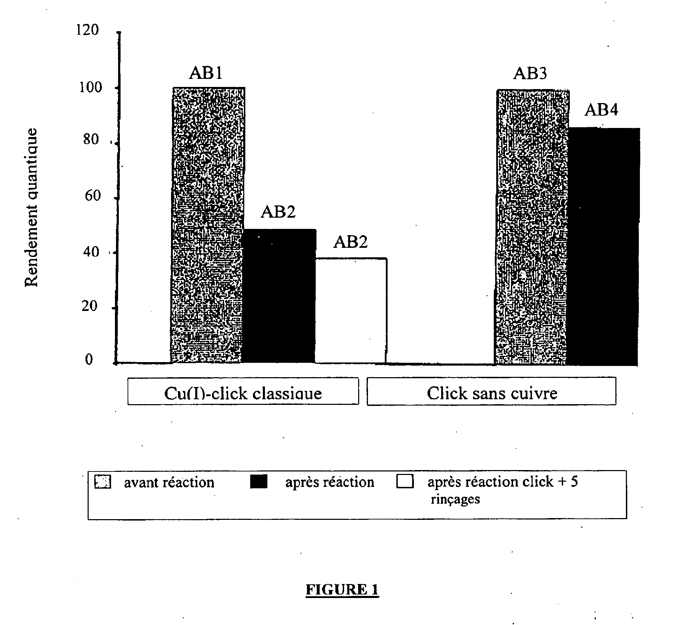

La figure 1 rend compte des rendements quantiques des QDots modifiés par *click chemistry.*

De plus, le quenching dû à la présence de Cu(I) est irréversible : même après plusieurs cycles de filtration sur filtre Microcon®/rinçage au PBS, le rendement quantique du produit AB2 ne revient pas au niveau de celui du produit de départ (ce dernier diminue même du fait de ce traitement).

**Exemple 3 :**

**Fonctionnalisation de lames de verre**

Les Qdots-cyclooctynes synthétisés tels que décrit dans l'exemple 1 sont utilisés pour réaliser une réaction de cycloaddition sur surface de verre fonctionnalisée par des azotures.

Des gouttes de la solution de QDots de départ et de QDot-cyclooctynes sont incubées à température ambiante dans le PBS pendant 1h, puis les lames sont rincées et les lames observées au scanner GeneTacIV sous excitation à 488 nm. Peu de signal non spécifique est observé (sédimentation partielle des nanoparticules) alors que le signal spécifique est nettement plus intense et dépendant de la concentration.

**Exemple 4:**

**Marquage cellulaire *in vivo***

Les QDots-cyclooctynes synthétisés tels que décrit dans l'exemple 1 sont utilisés pour réaliser un marquage cellulaire. Le but est d'imager l'incorporation métabolique d'acides sialiques modifiés par des azotures dans les surfaces membranaires de cellules CHO en culture.

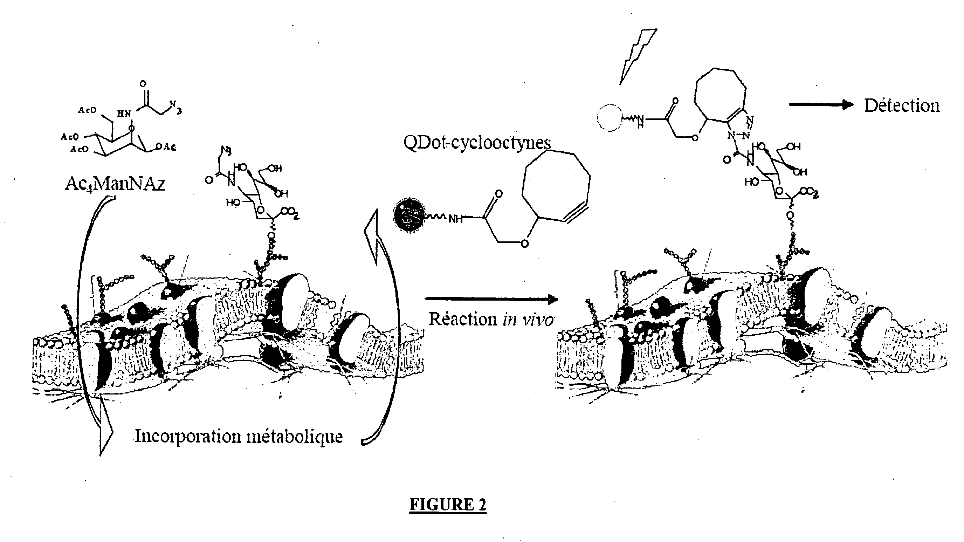

La figure 2 illustre le principe de l'incorporation métabolique de l'Ac$_4$ManNAz et sa transformation en acide sialique porteur du groupe N$_3$, lequel se trouve ensuite incorporé dans les protéoglycanes exprimés sur les membranes cellulaires et permet le marquage dans une deuxième étape par une réaction click avec les QDots-cyclooctynes.

L'incorporation d'une mannosamine modifiée par l'azoture (Ac$_4$ManNAz) pendant 3 jours est révélée ensuite par incubation pendant 4h à 4 °C avec la sonde QDot-cyclooctynes, puis observée par microscopie de fluorescence.

Un témoin négatif est obtenu par incorporation identique d'une mannosamine ne portant pas la modification azoture (Ac$_4$ManNAc). Les noyaux des cellules ont été marqués par le DAPI. Un marquage très net des membranes est visible dans le cas de l'incubation avec les Qdot-cyclooctynes permettant de valider l'utilisation de tels objets pour l'imagerie.

**Revendications**

1. Procédé utile pour modifier en surface un nanocristal semi-conducteur comprenant au moins les étapes consistant à :

   - disposer d'un nanocristal semi-conducteur dont la couche de revêtement organique est pourvue, en surface externe du nanocristal, d'au moins un groupement G1 réactif selon une réaction de cycloaddition de type *click chemistry,* et
   - mettre en présence ledit nanocristal avec un matériau annexe pourvu en surface d'au moins un groupement G2 complémentaire du groupement G1 au regard de ladite réaction *click chemistry*, dans des conditions propices à l'interaction desdits groupements G1 et G2,
   **caractérisé en ce que** lesdits groupements G1 et G2 sont respectivement un azoture et un radical cycloalcynyle contraint, ou inversement.

2. Procédé selon la revendication précédente, dans lequel la couche de revêtement organique dudit nanocristal semi-conducteur comprend au moins un ligand spécifique répondant à la formule générale (I) :

   (I)     L-X-E-A-G

   dans laquelle :

   - L est un ligand chélatant,
   - E est un groupe espaceur organique,
   - X est le produit réactionnel d'une fonction réactive présente sur le ligand L avec une fonction réactive complémentaire présente sur le groupe espaceur organique de deux fonctions réactives,
   - A représente une liaison simple ou un groupe choisi parmi -CONH-, -NHCO-, -OCH$_2$CONH-, -NHCOCH$_2$O-, -O-, -S-, et
   - et
   - G est un radical azoture ou cycloalcynyle contraint.

3. Procédé selon la revendication 1 ou 2, dans lequel le groupement G1 présent sur la couche de revêtement organique dudit nanocristal semi-conducteur est un radical cycloalcynyle contraint.

4. Procédé selon l'une quelconque des revendications précédentes, dans lequel le radical cycloalcynyle contraint est un radical cyclooctynyl.

5. Procédé selon l'une quelconque des revendications précédentes, dans lequel ledit nanocristal semi-conducteur a une structure coeur/coquille(s), ledit coeur étant constitué par un nanocristal constitué d'au moins un métal et/ou au moins un composé semi-conducteur tandis que la(les) coquille(s) est(sont) chacune constituée(s) d'une couche d'un métal et/ou d'au moins un composé semi-conducteur comprenant au moins un métal.

6. Procédé selon la revendication précédente, dans lequel le coeur est en un premier composé semi-conducteur de type A(II)B(VI) dans lequel A représente un métal ou un métalloïde à l'état d'oxydation +II et B représente un élément à l'état d'oxydation -II, tel que CdSe ou un composé C(III) D(V) dans lequel C représente un métal ou un métalloïde à l'état d'oxydation +III et D représente un élément à l'état d'oxydation -III tel que InP, tandis que la coquille entourant le coeur ou la première coquille entourant le coeur, est en un second composé semi-conducteur de type A(II)B(VI) différent du premier composé semi-conducteur, choisi parmi ZnSe, ZnS et CdS.

7. Procédé selon l'une quelconque des revendications précédentes, dans lequel la réaction est réalisée à température ambiante et en milieu aqueux.

8. Procédé selon l'une quelconque des revendications précédentes, dans lequel le matériau annexe est un substrat choisi parmi des surfaces de type métalliques, semi-conducteurs, oxydes, tels qu'alumines, $SiO_2$, $TiO_2$ et organiques.

9. Procédé selon l'une quelconque des revendications 1 à 7, dans lequel le matériau annexe est une molécule d'intérêt.

10. Procédé selon la revendication précédente, dans lequel la molécule d'intérêt est choisie parmi les marqueurs, les colorants, les fluorophores les linkers, les actifs thérapeutiques, cosmétiques, phytosanitaires, les membres d'une paire liante spécifique, les peptides, les aminoacides, cosmétiques, phytosanitaires, les membres d'une paire liante spécifique, les peptides, les aminoacides et les résidus d'aminoacide, les polypeptides, les sucres et les résidus du sucre, les photosensibilisateurs, les porphyrines des composés présentant une isomérie cis-trans tels que les dérivés des diaryléthylènes, des spiropyrannes, des spiroxazines, des fulgides ou de l'azobenzène.

11. Nanocristal semi-conducteur dont la couche de revêtement organique est pourvue, en surface externe du nanocristal, d'au moins un radical cycloalcynyle contraint et réactif à l'égard d'un azoture selon une réaction de cycloaddition de type *click chemistry*.

12. Nanocristal semi-conducteur selon la revendication précédente, dont la couche de revêtement de solubilisation comprend au moins un ligand spécifique répondant à la formule générale (I) :

(I)     L-X-E-A-G

dans laquelle :

- L est un ligand chélatant,
- E est un groupe espaceur organique,
- X est le produit réactionnel d'une fonction réactive présente sur le ligand L avec une fonction réactive complémentaire présente sur le groupe espaceur organique,
- A représente une liaison, simple ou un groupe choisi parmi -CONH-, -NHCO-, -OCH$_2$CONH-, -NHCOCH$_2$O-, -O-, -S-, et
- G est un radical cycloalcynyle contraint.

13. Nanocristal selon la revendication 11 ou 12, dans lequel le radical cycloalcynyle contraint est un radical cyclooctynyle.

**Patentansprüche**

1. Verfahren geeignet für die Modifizierung der Oberfläche eines Halbleitemanokristalls, umfassend mindestens die Schritte, die darin bestehen:

- einen Halbleiternanokristall bereitzustellen, dessen organische Überzugsschicht auf der äußeren Oberfläche des Nanokristalls mit mindestens einer Gruppierung G1 versehen ist, die bezüglich einer Cycloadditionsreaktion vom *Click-Chemie*-Typ reaktiv ist,
und
- den genannten Nanokristall mit einem zu bindenden Material, das auf der Oberfläche des Nanokristalls mit mindestens einer Gruppierung G2 versehen ist, die bezüglich der genannten *Click-Chemie*-Reaktion komplementär ist, bei für die Wechselwirkung der genannten Gruppierungen G1 und G2 geeigneten Bedingungen in Kontakt bringt, **dadurch gekennzeichnet, dass** die genannten Gruppierungen G1 und G2 ein Azid bzw. ein gespannter Cycloalkinylrest, oder umgekehrt, sind.

2. Verfahren nach dem vorhergehenden Anspruch, worin die organische Überzugsschicht des genannten Halbleiternanokristalls mindestens einen spezifischen Liganden umfasst, der der allgemeinen Formel (I) entspricht:

(I)         L-X-E-A-G

in der:

- L ein Chelat bildender Ligand ist,
- E eine organische Abstandsgruppe ist,
- X das Reaktionsprodukt ist einer reaktiven Funktion, die auf dem Liganden L vorhanden ist, mit einer komplementären reaktiven Funktion, die auf der organischen Abstandsgruppe der beiden reaktiven Funktionen vorhanden ist,
- A eine Einfachbindung oder eine Gruppe ausgewählt aus -CONH-, -NHCO-, OCH$_2$CONH-, -NHCOCH$_2$O-, -O-, -S- ist
- und
- G ein Aziderst oder einen gespannten Cycloalkinylrest bedeutet.

3. Verfahren nach Anspruch 1 oder 2, worin die Gruppe G1, die auf der organischen Überzugsschicht des genannten Halbleiternanokristalls vorhanden ist, ein gespannter Cycloalkinylrest ist.

4. Verfahren nach einem der vorhergehenden Ansprüche, worin der gespannte Cycloalkinylrest ein Cyclooctinylrest ist.

5. Verfahren nach einem der vorhergehenden Ansprüche, worin der Halbleitemanokristall eine Kern/Schale(n)-Struktur aufweist, wobei der Kern aus einem Nanokristall gebildet ist, der aus mindestens einem Metall und/oder mindestens einer Halbleiterverbindung gebildet ist, während die Schale(n) jeweils aus einer Schicht aus einem Metall und/oder mindestens einer Halbleiterverbindung, die mindestens ein Metall umfasst, gebildet ist (sind).

6. Verfahren nach dem vorhergehenden Anspruch, worin der Kern aus einer ersten Halbleiterverbindung des Typs A(II)B(VI), worin A ein Metall oder ein Metalloid im Oxidationszustand +II und B ein Element im Oxidationszustand -II bedeuten, wie CdSe, oder aus einer Verbindung C(III) D(V), worin C ein Metall oder ein Metalloid im Oxidationszustand +III und D ein Element im Oxidationszustand -III bedeuten, wie InP, gebildet ist, während die Schale, die den Kern oder die erste den Kern umhüllende Schale umgibt, aus einer zweiten Halbleiterverbindung des Typs A(II)B(VI), die von der ersten Halbleiterverbindung verschieden ist, gebildet ist, ausgewählt aus ZnSe, ZnS und CdS.

7. Verfahren nach einem der vorhergehenden Ansprüche, worin die Reaktion bei Raumtemperatur in wässrigem Medium durchgeführt wird.

8. Verfahren nach einem der vorhergehenden Ansprüche, worin das zu bindende Material ein Substrat ist ausgewählt aus Oberflächen des metallischen, halbleitenden, oxidischen, wie aluminiumoxidischen, SiO$_2$, TiO$_2$ und organischen Typs.

9. Verfahren nach einem der Ansprüche 1 bis 7, worin das zu bindende Material ein interessierendes Molekül ist.

10. Verfahren nach dem vorhergehenden Anspruch, worin das interessierende Molekül ausgewählt ist aus Markern, Farbstoffen, Fluorophoren, Linkerverbindungen, therapeutischen Wirkstoffen, Kosmetika, Pflanzenschutzmitteln, Partnern eines spezifischen Bindungspaares, Peptiden, Aminosäuren, Kosmetika, Pflanzenschutzmitteln, Partnern eines spezifischen Bindungspaares, Peptiden, Aminosäuren und Aminosäureresten, Polypeptiden, Zuckern und Zuckerresten, Photosensibilisatoren, Porphyrinen, Verbindungen mit einer cis-trans-Isomerie, wie Diarylethylenderivaten, Spiropyranen, Spiroxazinen, Fulgiden oder Azobenzol.

11. Halbleitemanokristall, dessen organische Überzugsschicht auf der äußeren Oberfläche des Nanokristalls mit mindestens einem gespannten Cycloalkinylrest versehen ist und der bezüglich einer Cycloadditionsreaktion vom *Click-Chemie*-Typ mit einem Azid reaktiv ist.

12. Halbleiternanokristall nach dem vorhergehenden Anspruch, worin die löslichmachende Überzugsschicht mindestens einen spezifischen Liganden umfasst, der der allgemeinen Formel (I) entspricht:

(I)      L-X-E-A-G

in der:

- L ein Chelat bildender Ligand ist,
- E eine organische Abstandsgruppe ist,
- X das Reaktionsprodukt ist einer reaktiven Funktion, die auf dem Liganden L vorhanden ist, mit einer komplementären reaktiven Funktion, die auf der organischen Abstandsgruppe der beiden reaktiven Funktionen vorhanden ist,
- A eine Einfachbindung oder eine Gruppe ausgewählt aus -CONH-, -NHCO-, - OCH$_2$CONH-, -NHCOCH$_2$O-, -O-, -S- ist und
- G ein Azidrest oder einen gespannten Cycloalkinylrest bedeutet.

13. Nanokristall nach Anspruch 11 oder 12, worin der gespannte Cycloalkinylrest ein Cyclooctinylrest ist.

**Claims**

1. Method useful for - surface-modifying a semiconductor nanocrystal comprising at least the steps consisting in:

- having a semiconductor nanocrystal, the organic coating layer of which is provided, at the outer surface of the nanocrystal, with at least one reactive group G1 that reacts according to a cycloaddition reaction of *click chemistry* type; and
- bringing said nanocrystal together with an adjoining material provided at the surface with at least one G2 group complementary to the G1 group with respect to said *click chemistry* reaction, under conditions favourable to the interaction of said G1 and G2 groups,
**characterized in that** said G1 and G2 groups are respectively an azide and a strained cycloalkynyl radical, or vice versa.

2. Method according to the preceding claim, wherein the organic coating layer of said semiconductor nanocrystal comprises at least one specific ligand corresponding to the general formula (I):

(I)      L-X-E-A-G

in which:

- L is a chelating ligand;
- E is an organic spacer group;
- X is the reaction product of one reactive functional group present on the ligand L with a complementary reactive functional group present on the organic spacer group of two reactive functional groups;
- A represents a single bond or a group chosen from the group consisting of -CONH, -NHCO-,-OCH$_2$CONH-, -NHCOCH$_2$O-, -O-, -S-; and
- G is a strained cycloalkynyl or azide radical.

3. Method according to claim 1 or 2, wherein the G1 group present on the organic coating layer of said semiconductor nanocrystal is a strained cycloalkynyl radical.

4. Method according to any one of the preceding claims, wherein the strained cycloalkynyl radical is a cyclooctynyl radical.

5. Method according to any one of the preceding claims, wherein said semiconductor nanocrystal has a corelshell(s) structure, said core being constituted by a nanocrystal constituted of at least one metal and/or at least one semiconductor compound whilst the shell(s) is (are) each constituted of a layer of a metal and/or of at least one semiconductor compound comprising at least one metal.

6. Method according to the preceding claim, wherein the core is made from a first semiconductor compound of A(II)B(VI) type, in which A represents a metal or a metalloid in the +II oxidation state and B represents an element in the -II oxidation state such as CdSe or a C(III) D(V) compound in which C represents a metal or a metalloid in the +III oxidation state and D represents an element in the -III oxidation state such as InP, whilst the shell surrounding the core or the first shell surrounding the core is made from a second semiconductor compound of A(II)B(VI) type different from the first semiconductor compound, chosen from the group consisting of ZnSe, ZnS and CdS.

7. Method according to any one of the preceding claims, wherein the reaction is carried out at ambient temperature and in an aqueous medium.

8. Method according to any one of the preceding claims, wherein the adjoining material is a substrate chosen from surfaces of the group consisting of metallic type, semiconductor, oxides, such as aluminas, $SiO_2$, $TiO_2$ and organic type,

9. Method according to any one of claims 1 to 7, wherein the adjoining material is a molecule of interest.

10. Method according to the preceding claim, wherein the molecule of interest is chosen from the group consisting of markers, dyes, fluorophores, linkers, therapeutic active agents, cosmetic active agents, phytosanitary active agents, members of a specific binding pair, peptides, amino acids, cosmetics, phytosanitary, members of a specific binding pair, peptides, amino acids, and amino acid residues, polypeptides, sugars and sugar residues, photosensitizers, porphyrins, compounds that have cis-trans isomerism such as derivatives of diarylethylenes, spiropyrans, spirooxazines, fulgides or azobenzene.

11. Semiconductor nanocrystal, the organic coating layer of which is provided, on the outer surface of the nanocrystal, with at least one strained cycloalkynyl radical that is reactive with regard to an azide according to a cycloaddition reaction of *click chemistry* type.

12. Semiconductor nanocrystal according to the preceding claim, the solubilisation coating layer of which comprises at least one specific ligand corresponding to the general formula (I):

(I)        L-X-E-A-G

in which:

- L is a chelating ligand;
- E is an organic spacer group;
- X is the reaction product of one reactive functional group present on the ligand L with a complementary reactive functional group present on the organic spacer group;
- A represents a single bond or a group chosen from the group consisting of -CONH, -NHCO-,-OCH$_2$CONH-, -NHCOCH$_2$O-, -O-, -S-; and
- G is a strained cycloalkynyl radical.

13. Nanocrystal according to claim 11 or 12, wherein the strained cycloalkynyl radical is a cyclooctynyl radical.

**FIGURE 1**

Ac₄ManNAz

QDot-cyclooctynes

Détection

Incorporation métabolique

Réaction *in vivo*

**FIGURE 2**

## RÉFÉRENCES CITÉES DANS LA DESCRIPTION

**Documents brevets cités dans la description**

- WO 2006050262 A

**Littérature non-brevet citée dans la description**

- **VUNDYALA et al.** *Tetrahedron Letters,* 2008, vol. 29, 6386

- **BINDER, W. H. ; SACHSENHOFER, R. ; STRAIF, C. J. ; ZIRBS, R.** *Journal of Materials Chemistry,* 2007, vol. 17 (20), 2125-2132